# EUROPEAN PATENT APPLICATION

(11) **EP 1 067 141 A1**
(43) Date of publication of application: **10.01.2001**
(21) Application number: 99201864.8
(22) Date of filing: 11.06.1999
(51) Int. Cl.: C07K 14/47, C07K 16/18, C12N 5/08, A61K 38/16, G01N 33/68, A61P 37/06

(54) **A male-specific protein homologue involved in histocompatibility**

(71) Applicant: Rijksuniversiteit te Leiden, 2312 AV Leiden (NL)
(72) Inventor: The designation of the inventor has not yet been filed
(74) Representative: Ottevangers, Sietse Ulbe

(57) **Abstract**

Graft rejection and graft versus host disease after HLA-identical stem cell transplantation is the result of recognition of minor histocompatibility antigens on donor stem cells by immunocompetent T lymphocytes from recipient origin. T lymphocyte clones specifically recognizing H-Y epitopes on male target cells have been generated during graft-rejection after sex-mismatched transplantation. Previously, two human H-Y epitopes derived from the same SMCY gene have been identified that were involved in bone marrow graft rejection. Here, we report the identification of a new male-specific transplantation antigen encoded by the Y-chromosome-specific gene DFFRY. The DFFRY-derived peptide was recognized by a HLA-A1 restricted CTL clone, which was generated during graft rejection from a female patient with acute myeloid leukemia who rejected her HLA-phenotypically identical bone marrow from her father. The identification of this gene demonstrates that at least two genes present on the human Y-chromosome code for male-specific transplantation antigens.

## Description

The present invention relates to the field of immunology, in particular to the field of cellular immunology.

It is also concerned with the areas of organ transplantation, grafting of tissues and/or cells, especially bone marrow and possible immunological reactions caused by transplantation and/or grafting and blood transfusion.

Since the invention concerns a sex-related proteinaceous material, encoded in nature by a sex-related homologue of a gene, the invention also relates to the areas of sex linked congenital aberrations, of embryonic selection techniques, in vitro fertilisation techniques, vaccination and in ovo vaccination.

Bone marrow transplantation (BMT), one of the areas the invention is concerned with and the area from which the present invention originates, finds its application in the treatment of for instance severe aplastic anaemia, leukaemia and immune deficiency diseases.

In the early days of this technique many transplants failed through rejection of the graft by the host. Transplants that did succeed, however often led to an immune response by lymphocytes present in the graft against various tissues of the host (Graft versus Host Disease (GvHD)).It is now known that the GvHD response is mainly due to the presence of major H antigens which present a transplantation barrier. Therefor it is now routine practice to graft only HLA-matched materials (either from siblings or unrelated individuals) resulting in a much improved rate of success in bone marrow transplantation. However, despite this improvement, as well as improvements in pretransplantation chemotherapy or radiotherapy and the availability of potent immunosuppressive drugs, about 20-70% of the treated patients still suffer from GvHD (the percentage is age and bone marrow donor dependent). To avoid GvHD it has been suggested to remove the cells (mature T cells) causing said reaction from the graft. This however often leads to graft failure or to recurrence of the original disease. The cells responsible for GvHD are also the cells which often react against the original aberrant cells in for instance leukaemia (Graft versus Leukaemia response).

Since BMT is nowadays only carried out with HLA matched grafts, the GvHD and/or rejections which still occur must be caused by another group of antigens. Both complications are thought to be initiated by HLA-restricted T-lymphocytes that recognize minor histocompatibility antigens (1-4). It is thought very likely that this group of so called minor H antigens (mHag), which are non-MHC encoded histocompatibility antigens (unlike the major H antigens) are at least partially responsible for the remaining incidence of GvHD and/or rejection.

mHag's have originally been discovered in congeneic strains of mice in tumor rejection and skin rejection studies. In mice, the use of inbred strains has shown that mHag are encoded by almost 50 different allelically polymorphic loci scattered throughout the genome. In humans, mHag have been shown to exist, although their overall number and complexity remains uncertain.

These polymorphic mH antigens which differ between donor and recipient are peptides derived from intracellular proteins. The male-specific H-Y antigens are the most extensively studied mH antigens. The involvement of H-Y antigens in transplant rejection was identified by the observation that within an inbred mouse strain females rapidly rejected primary syngeneic male skin grafts (5,6). In humans, female patients transplanted with male bone marrow had a higher risk of developing graft rejection than when transplanted with marrow of female origin (1,7-9). In both mice and humans, H-Y specific T lymphocyte clones could be generated from peripheral blood of patients during GVHD or graft rejection after sex-mismatched transplantation (1,10,11). These H-Y specific T lymphocyte clones can be used as tools to identify H-Y antigens and the corresponding Y-specific genes.

The first identified gene coding for a H-Y antigen was SMCY. In mouse, the gene was identified by transfection of a series of cosmids of the short arm of the Y chromosome into cells expressing the appropriate restriction molecules. These cells were then tested for their ability to stimulate a H-Y epitope-specific T lymphocyte clone. Transfection of one cosmid coding for the SMCY gene resulted in stimulation of the H-Y specific T lymphocyte clone (10). In humans, two H-Y antigens were identified by analysis of eluted peptides from male target cells using a combination of microcapillary liquid chromatography-electrospray ionization mass spectrometry with T cell epitope reconstitution assays. Both H-Y antigens, recognised by a H-Y specific HLA-B7 restricted cytotoxic T lymphocyte (CTL) clone and a HLA-A2 restricted CTL clone, were encoded by different peptides from the SMCY protein (12,13).

Although SMCY encodes for the majority of H-Y antigens identified to date, genetic mapping of the mouse Y chromosome suggested that at least two and up to five distinct loci, including SMCY, encode H-Y antigens (14). Recently, a systematic search of the non-recombining region of the human Y chromosome identified three known and five novel Y-specific genes with a ubiquitous tissue expression (15). Each gene had a homologue on the X chromosome encoding a very similar but nonidentical protein isoform. The amino acid sequence identity of the X-Y isoforms varied from 85% to 97%. The identification of UTY as a mouse H-Y antigen encoding gene confirmed the hypothesis that H-Y antigen is the product of more than one gene on the Y-chromosome in mice(16). In humans however, the only known H-Y antigens are derived from the SMCY gene (12,13).

In 1990, we reported a detailed analysis of a female patient with acute myeloid leukemia (AML) who rejected a bone marrow graft from her HLA-phenotypically identical father. Before transplantation and during graft rejection, a strong cellular recipient anti-donor cytotoxic reactivity could be demonstrated. This reactivity was directed against mH antigens, including a male specific antigen. Cloning of this recipient-anti-donor response resulted in the generation of a CTL clone which was shown to be H-Y specific and HLA-A1 restricted (1).

The present invention provides the identification of a novel male-specific transplantation antigen, recognised by such a HLA-A1 restricted CTL clone, showing for the first time that more genes may be involved in male specific antigenicity. Thus the invention provides DFFRY or DFFRX proteins or homologues, derivatives and/or specific fragments thereof for use as a pharmaceutical, in particular a medicament for modifying an immune response in a host. As will be shown herein, these polymorphic proteins are different between males and females and lead to peptides that can elicit a T cell response in the other sex. The result has been achieved in man, but other mammals and possibly other vertebrates may also have the same polymorphic homologues of the human DFFRY/X proteins, which are also part of this invention. Polymorphic variants and other derivatives (having e.g. conserved amino acid substitutions or deletions of irrelevant sites) are also part of the present invention. A very important part of the present invention are antigenic peptides, in particular those eliciting T cell responses. Methods for identifying T cell epitopes in a protein such as DFFRY/X are known in the field. They include computerised searches for anchor sites, processing sites, etc., as well as binding studies on empty MHC molecules and the capability to stimulate T cells. These peptides can be derivatised for increasing their capability to modify immune responses by randomising certain residues and studying the effect in assays. Techniques such as phage display and modern methods of pepscan can be used in this respect. The molecules according to the invention are useful for modifying immune responses in a host, typically in a setting for transplantation of tissue and/or cells. Modifying may be either enhancing or reducing depending on the application and host selected. More details will be given below. Thus in a further embodiment the invention provides the use of DFFRY or DFFRX proteins or homologues, derivatives and/or specific fragments thereof in the preparation of a medicament for modifying an immune response in a host.

As said before, in one embodiment the molecules are used for modifying an immune response in a host. This host may be a donor of cells and/or tissue, or a recipient of cells and/or tissue. The modification may be e.g. inducing tolerance to the molecules of the invention.

In a preferred embodiment the cells to be transferred from donor to recipient are hematopoietic cells from any suitable source. Thus the invention also provides the use of the invented molecules wherein donor cells comprise hematopoietic cells.

Suitable sources of hematopoietic cells include bone marrow, fetal liver cells and umbillical cord blood. The invention further provides a use of the invented molecules wherein at least part of said donor cells constitute an organ allograft. Desired organs to be transplanted from donor to recipient, or from artificial production system to recipient are well known in the art. In order to do bone marrow, cord blood, organ or any other transplantations, matching must be carried out and for the extent it cannot be carried out, tolerance may be induced. Ways of inducing tolerance in hosts donors and/or recipients) are given below. Thus the invention also includes a use according to the invention, wherein a medicament comprising at least one molecule according to the invention induces tolerance for DFFRY or DFFRX proteins or homologues, derivatives and/or specific fragments thereof in said host.

In yet another embodiment one can avoid adverse effects of immunological recognition of molecules of the invention by at least partial elimination of said response. This is also part of the present invention. Thus the invention provides a use of a medicament whereby said medicament at least partially eliminates an immune response to DFFRY or DFFRX proteins or homologues, derivatives and/or specific fragments thereof in said host, in particular whereby said medicament eliminates a subset of T cells. In order to be able to match, one must be able to type the set of major and minor histocaompatibility antigens of a host. Thus the invention provides a diagnostic testkit for HLA matching and/or mismatching between at least two individuals, comprising a means for determining the presence or absence of DFFRY or DFFRX proteins or homologues, derivatives and/or specific fragments thereof in a sample. Testing can be done both at the protein level and at the nucleic acid level. Therefore the invention also provides a diagnostic testkit for HLA matching and/or mismatching between at least two individuals, comprising a means for determining the presence or absence of nucleic acids encoding DFFRY or DFFRX proteins or homologues, derivatives and/or specific fragments thereof in a sample. The testkits can be produced in the same manner as testkits for other mHAg's and are well known in the art. They include antibody/antigen based interactions and the like. They also include PCR and other amplification reactions and other reactions based on hybridisation, etc.

In a preferred embodiment the invention provides a peptide constituting a T-cell epitope obtainable from a DFFRY or DFFRX protein or homologue thereof, in particular a peptide constituting a T-cell epitope obtainable from a DFFRY or DFFRX protein or homologue thereof comprising the sequence IVDCLTEMY or IVDCLTEMYY or a derivative thereof having similar immunological properties, as well as an immunogenic polypeptide obtainable from a DFFRY or DFFRX protein or homologue thereof comprising the sequence IVDCLTEMY or IVDSLTEMY or IVDCLTEMYY or IVDSLTEMYY or a derivative thereof having similar immunological properties.

The way these sequences are obtained is described herein. An important part of this novel method of arriving at said sequences is the purification and the choice of the starting material. Said novel method is therefor also part of the scope of this invention. However, now that the sequence is known, it is of course no longer necessary to follow that method, because the peptides can easily be made synthetically, as is well known in the art. Since routine techniques are available for producing synthetic peptides, it is also within the skill of the art to arrive at analogs or derivatives of the explicitly described peptides, which analogs and/or derivatives may have the same or at least similar properties and or activity. On the other hand analogs which counteract the activity of the explicitly described peptides are also within the skill of the art, given the teaching of the present invention. Therefor derivatives and/or analogs, be it of the same or different length, be it agonist or antagonist, be it peptide-like or peptidomimetic, are part of the scope of this invention.

A preferred embodiment of the present invention is a peptide with the sequence IVDCLTEMY(Y). This does not imply that other peptides are not suitable. This will for a large part depend on the application and on other properties of the peptides, which were not all testable within the scope of the present invention.

The peptides and other molecules according to the invention find at least part of their utility in that they may be used to induce tolerance of the donor immune system in DFFRY/X negative donors, so that residual peripheral blood lymphocytes in the eventually transplanted organ or the bone marrow, as it may be do not respond to host DFFRY/X material in an DFFRY/X positive recipient. In this way GvHD may be prevented. On the other hand tolerance may be induced in DFFRY/X negative recipients in basically the same way, so that upon receipt of an organ or bone marrow from an DFFRY/X positive donor no rejection on the basis of the DFFRY/X material occurs.

For tolerance induction very small doses can be given repeatedly, for instance intravenously, but other routes of administration may very well be suitable too. Another possibility is the repeated oral administration of high doses of the peptides. The peptides may be given alone, or in combination with other peptides, or as part of larger molecules, or coupled to carrier materials in any suitable excipients.

Further applications of the peptide or derivatives thereof lie in the prophylactic administration of such to transplanted individuals to prevent GvHD. This can be done with either agonists, possibly in combination with an adjuvant, or with antagonists which may block the responsible cells. This can be done with or without the concomittant administration of cytokines.

Furthermore the peptides or antibodies thereto can be used in so called "magic bullet" applications, whereby the peptide or the antibody is coupled to a toxic substance to eliminate certain subsets of cells.

Diagnostic applications are clearly within the skill of the art. They include, but are not limited to H-Y typing, detection of genetic aberrancies and the like.

Other therapeutical applications of the peptide include the induction of tolerance to DFFRY/X proteins in DFFRY/X related (auto)immune diseases, such as possibly in Rheumatoid arthritis. On the other hand they may be used in vaccines in DFFRY/X related (auto)immune diseases.

For the sake of illustration a number of applications is cited below.

The DFFRY/X peptide or its derivatives can be used to prevent harmful reaction of the recipient towards the donor or vice versa; in all forms of transplantation i.e. organs, tissues and bone marrow. Assuming that residual donor peripheral blood lymphocytes (PBL)'s in the transplanted organ could react with and/or against host PBL's and even could cause GvHD, the DFFRY/X peptide could be used to induce tolerance in living organ (kidney, liver, gut, skin) of DFFRY/X negative donors for DFFRY/X positive patients. In bone marrow transplantation, the DFFRY/X peptide (given alone or in combination with other (H-Y) peptides) can be used to induce tolerance in the living bone marrow donor. The peptide(s) can be given orally, intravenous or otherwise.

In all forms of organ (including cornea), tissue (including heartvalves and skin) and bone marrow transplantation with living or cadaveric donors, the DFFRY/X peptide could be used to induce tolerance in DFFRY/X negative recipients of organ and tissue transplants from DFFRY/X positive donors. In case of bone marrow transplantation, tolerance must be induced in female donors for male recipients. The tolerance induction can be achieved by clinical application of the DFFRY/X peptide systematically, i.v., locally, orally, as eye-drops.

The DFFRY/X peptide could act in a non-allelic restricted manner (thus promiscuous) implicating that its applicability to inducing tolerance is not restricted to the HLA type of the (fe)male donors and (fe)male recipients and donors .

The DFFRY/X peptide or its derivatives can be applied to generate reagents and/or medicine.As Graft-versus-Host disease and rejection prophylaxis administration to the transplanted individual either with or without adjuvant of
a) the DFFRY/X peptide
b) DFFRY/X peptide analogues, including left or right turning peptides
c) DFFRY/X peptide antagonists

Usage of the DFFRY/X sequence information to generate, for immunomodulatory purposes:
a) anti-idiotypic T cells
b) anti-idiotypic B cells
c) human monoclonal antibodies

The DFFRY/X peptide or its derivatives can be used as a marker for sex linked congenital or other diseases.

It can be used for the generation of a genetic probe enabling screening for the congenital sex-linked disorders.

The genetic probe can be used for genetic counseling, population genetics and pre-natal diagnostic.

The defect can be repaired by genetic engineering.

The peptides and other molecules according to the invention can also be used for the production of anti-conceptive drugs

Furthermore the peptides and other molecules according to the invention can be used for the production of cytotoxic T lymphoctes (CTL) with specificity for the DFFRY/X sequence.

The DFFRY/X specific CTL can be used for selection of male embryos in X linked recessive disorders.

The invented molecules can be applied to generate reagents and/or medicine for
a) determination of foetal erythrocytes in maternal circulation.
b) intra uterine diagnostics.
c) use prior to implantation for in vitro fertilization.
d) determination of chimerism

Veterinary applications include:
a) embryonic selection.
b) in vitro fertilization.
c) vaccination and in ovo vaccination
d) anti-conception.

On the basis of the peptide/proteinaceosu molecule described herein genetic probes can be produced which can be used in detection kits. On the basis of the peptide described herein anti-idiotypic B cells and/ or T cells and antibodies can be produced. All these embodiments have been made possible by the present disclosure and therefor are part of the present invention.

The techniques to produce these embodiments are all within the skill of the art.

Dose ranges of peptides and antibodies and/or other molecules according to the invention to be used in the therapeutical applications as described herein before are usually designed on the basis of rising dose studies in the clinic. The doses for peptides may lie between about 0.1 and 1000 µg per kg body weight, preferably between 1 and 10 µg per kg body weight.

Thus the invention further provides a vaccine comprising an epitope or a polypeptide according to the invention, a pharmaceutical formulation comprising an epitope or a polypeptide according to the invention, a peptide or polypeptide according to the invention for use as a medicine, the use of a peptide or polypeptide according to the invention in the preparation of a medicament for the induction of tolerance for transplants to prevent rejection and/or Graft versus Host disease, a method for the elimination of a subset of T-cells recognizing a peptide according to the invention, wherein said peptide is provided with a toxic moiety, an analog of the peptide according to claim 12 or 13, which is an antagonist for the activity of T cells recognizing said peptide, as well as a method for the generation of antibodies, T cell receptors, anti-idiotypic B-cells or T-cells, comprising the step of immunisation of a mammal with a peptide or a polypeptide according to the invention and antibodies, T-cell receptors, B-cells or T-cells obtainable by said method.

### Detailed description.

### Materials and Methods

*CTL and cell lines.* The CD8+ CTL clone HLA-A1 HY was derived by limiting dilution from peripheral blood mononuclear cells (PBMC) of a female AML patient, who had rejected the phenotypically HLA-identical male bone marrow derived from her father (1). The CTL clone was cultured by stimulation with irradiated allogeneic PBMC and donor derived EBV-transformed B cells (EBV-LCL) in RPMI-1640 medium (Bio-Whittaker, Verviers, Belgium) containing 10% pooled human serum and IL-2 (Roussel Uclaf, Paris, France) at 300 IU/ml. As a control, a SMCY specific HLA-B7 restricted CTL clone was used (12). HeLa cells and WEHI-164 clone 13 cells were obtained from American Type Culture Collection, Rockville, MD and maintained in complete medium. The HLA-A1 gene was cloned into the vector PLXSN and stably transfected into HeLa (HeLa/HLA-A1) using retroviral transfection as is described by Millar et al. (17). After transfection, HeLa/HLA-A1 cells were maintained in complete culture medium supplemented with G418 (500 µg/ml).

*Cloning of Y-specific genes.* Total RNA was isolated from male EBV-LCL with Trizol (Gibco, BRL, Gaithersburg, MD) according to the manufacturer's procedure. cDNA was prepared from RNA using M-MLV BRL reverse transcriptase (Gibco, BRL) for 60 min. at 37°C as described (18,19). One fiftieth of each cDNA reaction was individually amplified using specific primers for each Y-gene (Table 1) identified by a systematic search of the nonrecombining region of the human Y chromosome. Each ubiquitously expressed gene had a homologue on the X chromosome encoding a very similar but nonidentical protein isoform. Bases indicated in bold are mutations compared to the original sequence of the Y-specific gene in order to introduce artificial start or stop codons or to introduce restriction sites for cloning experiments. Since both SMCY and DFFRY have a large open reading frame, each gene is cloned in three overlapping cDNA constructs. With the exception of SMCY, all genes were amplified using the expand long template PCR system (Boehringer Mannheim GmbH, Mannheim, Germany). The amplification was started with a denaturation step of 2 min. at 92°C, followed by 30 cycles, with each cycle consisting of 20 sec. at 92°C, 1 min. at 60°C and 1 min. at 68°C. SMCY was amplified using the Marathon cDNA amplification kit (Clontech Laboratories, Palo Alto, CA) according to the manufacturer's procedure. After amplification, each Y-specific cDNA was visualized on a ethidium bromide stained low melt agarose gel (Sigma Chemical, St. Louis, MI). cDNA was isolated from the low melt agarose gel using agarase (Boehringer Mannheim GmbH) according to the manufacturer's procedure. Each individual cDNA was cloned in the expression vector PCR3.1 using the eukaryotic TA cloning kit (Invitrogen Corporation, Carlsbad, CA).

*Cloning of subgenic fragments of the DFFRY2 gene*. Restriction analysis on the DFFRY2 gene revealed a XbaI site on position 3000 and a PstI site on position 4433 of the DFFRY cDNA. Both restriction endonucleases have also a recognition site in the multiple cloning site of the PCR3.1 plasmid directly after the 3' end of the DFFRY2 cDNA. Deletion mutants DFFRY2/XbaI and DFFRY2/PstI were generated by incubating the plasmid PCR3.1 containing the DFFRY2 gene with the restriction enzyme XbaI and PstI, deleting a 1877 bp and a 444 bp long fragment at the 3' site of the DFFRY2 gene respectively. After digestion, the linear truncated constructs were visualized on a ethidium bromide stained low melt agarose gel. After isolating the constructs with agarase, the linear constructs were ligated with the rapid DNA ligation kit (Boehringer Mannheim GmbH) to form circular plasmid DNA consisted of vector PCR3.1 with the truncated DFFRY2/XbaI and DFFRY2/PstI cDNAs.

*Cloning of DFFRY2 minigenes.* DFFRY2 minigenes were generated by hybridization of two oligo nucleotides, followed by incorporation into the BamHI and HindIII site of PCR3.1. For generation of minigene 14AA, we used the following oligo nucleotides (5'→3')

AGCTTCACCATGCTCAAACAGATAGTAGACTGTTTGACTGAAATGTATTACTAG and
GATCCTAGTAATACATTTCAGTCAAACAGTCTACTATCTGTTTGAGCATGGTGA.
For minigene 16AA we used the oligo nucleotides
AGCTTCACCATGAAACAGATAGTAGACTGTTTGACTGAAATGTATTACATGGGCACATAG
and
GATCCTATGTGCCCATGTAATACATTTCAGTCAAACAGTCTACTATCTGTTTCATGGTGA.

*Transfection of HeLa cells and sreening of transfectants*. Y-specific cDNA was transfected into HeLa cells by the DEAE-dextran-chloroquine method as described (20). One day before tranfection, HeLa cells were seeded in 96-well flat-bottom microtiter plates at 15,000 cells per well in 100 µl of complete culture medium. Before transfection, medium was discarded and replaced by 45 µl of DEAE-Dextran/DNA mixtures. These mixtures were prepared for duplicate transfections in 96-well V-bottom microtiter plates by sequentially adding: 98 µl of RPMI-1640 supplemented with 10% decomplemented NuSerum IV (Collaborative Biomedical Products, Bedford, MA) containing 0.3 mg/ml DEAE-dextran (Sigma Chemical) and 100 µM chloroquine, 1 µl of TE containing 200 ng Y-specific cDNA in plasmid PCR3.1 and 1 µl of TE (10 mM Tris, 1 mM EDTA, pH 7.4) containing 200 ng plasmid pcDNAI/AMP-A1 (plasmid pcDNAI/AMP containing the HLA-A1 gene) or no HLA-A1 construct as a control. If the stably HLA-A1 transduced HeLa/HLA-A1 cells were used no plasmid pcDNAI/AMP-A1 was added to the DEAE-dextran/DNA mixtures. The HeLa cells were incubated for 4 h at 37°C, after which the DEAE-dextran/DNA was discarded and replaced by 80 µl of PBS containing 10% DMSO. After 2 min. at room temperature, PBS-DMSO was replaced by 200 µl of complete culture medium. Transfected HeLa cells were incubated for 48 h at 37°C. After removing the medium, 4,000 HLA-A1 HY were added in 200 µl RPMI-1640 containing 10% pooled human serum and 300 IU IL-2/ml. After 24 hours, the TNF content in 50 µl supernatant was determined by adding it to 50 µl complete culture medium containing 50,000 WEHI-164 clone 13 cells, on which TNF has a cytolytic effect (21). After 18 hours, 10 µl cell proliferation reagent WST I (Boehringer Mannheim GmbH ) was added and the TNF concentration was calculated in comparison to recombinant TNF standards measured in the same assay.

*Protein synthesis.* The Y-specifc genes were transcribed and translated using Wheat Germ extract and ³H-labeled Leucine according to the manufacturer's procedure as is described in the TNT T7 coupled transcription/translation system (Promega Corporation, Madison, WI). The ³H-labeled proteins were separated on a 12% SDS PAGE gel for 2 hours. After fixation in a 10% methanol/10% glacial acetic acid solution for 30 min., amplification of radiation by the ³H-labeled proteins was achieved by using amplify fluorografhic reagent (Amersham). Then, the gel was dried for 2 hours at 65°C and the proteins were visualized by autoradiography. The molecular weights of the proteins were determined by using kaleidoscope prestained protein standards (Bio-Rad Laboratories, Hercules, CA).

*Peptide synthesis and peptide recognition assays*. Peptides were synthesized by solid-phase FMOC chemistry and Wang resins on an AMS 422 multiple peptide synthesizer (Gilson Medical Electronics, Middletown, WI) and characterized by mass spectrometry. Standard ⁵¹Cr-release assays were used to determine lysis of target cells. EBV-LCL target cells were labeled with 100 µCi of Na⁵¹CrO₄. After 1 hour of incubation at 37°C, the cells were washed three times with RPMI supplemented with 2% FCS. Then 2000 Na⁵¹CrO₄ labeled EBV-LCL cells/well were plated in a 96-wells V-bottom microtiter plate in 100µl RPMI-1640+10% pooled human serum containing various concentrations of peptide. After 1 hour of incubation at 37°C, 20,000 CTLs were added in 100 µl RPMI-1640+10% pooled human serum. ⁵¹Cr-release was measured after incubation at 37°C for 4 hours.

### Results

### Cloning of Y-specific genes

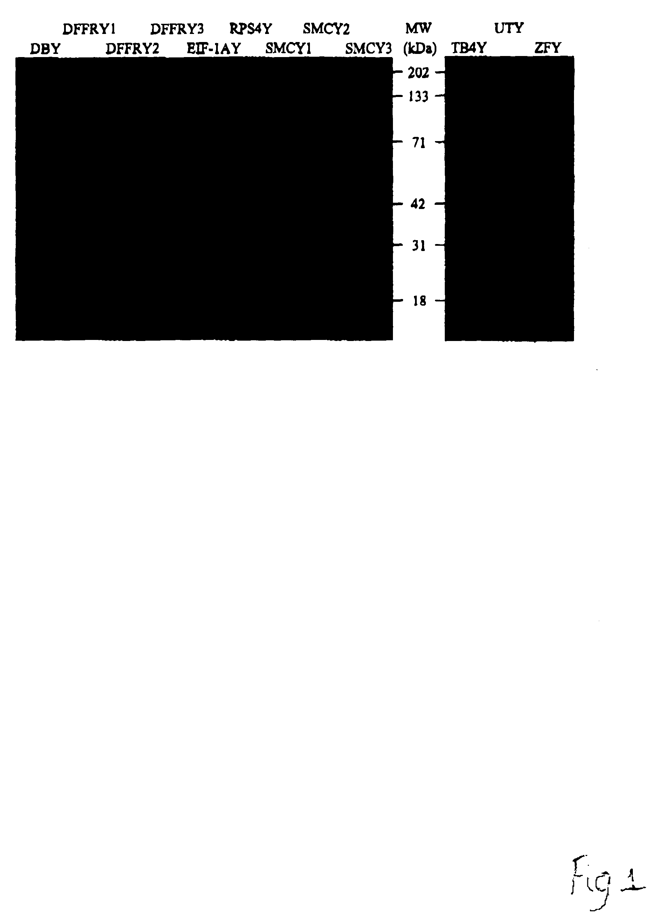

The PCR primers used as shown in table 1 were designed to amplify only the Y-specific gene and not the X-homologue. Restriction and sequence analysis of amplified Y-specific cDNAs confirmed the Y-specificity (data not shown). To determine whether the cloned Y-cDNAs were transcribed and translated appropriately, we performed an in vitro transcription/translation assay, in which the Y-specific proteins were labeled with ³H and visualized on an autoradiogram after separation on a SDS PAGE gel as shown in figure 1. All proteins showed the molecular weight (MW) as was deduced from their primary sequence, with the exception of ZFY. This protein had a MW of 125 kDa, whereas the sequence deduced MW was 90 kDa. However, DNA sequence analysis of both the 3' and 5' site of ZFY revealed the right ZFY DNA sequence, suggesting that this protein had undergone post-translational modification.

### Identification of the gene encoding for the HLA-A1 restricted H-Y epitope

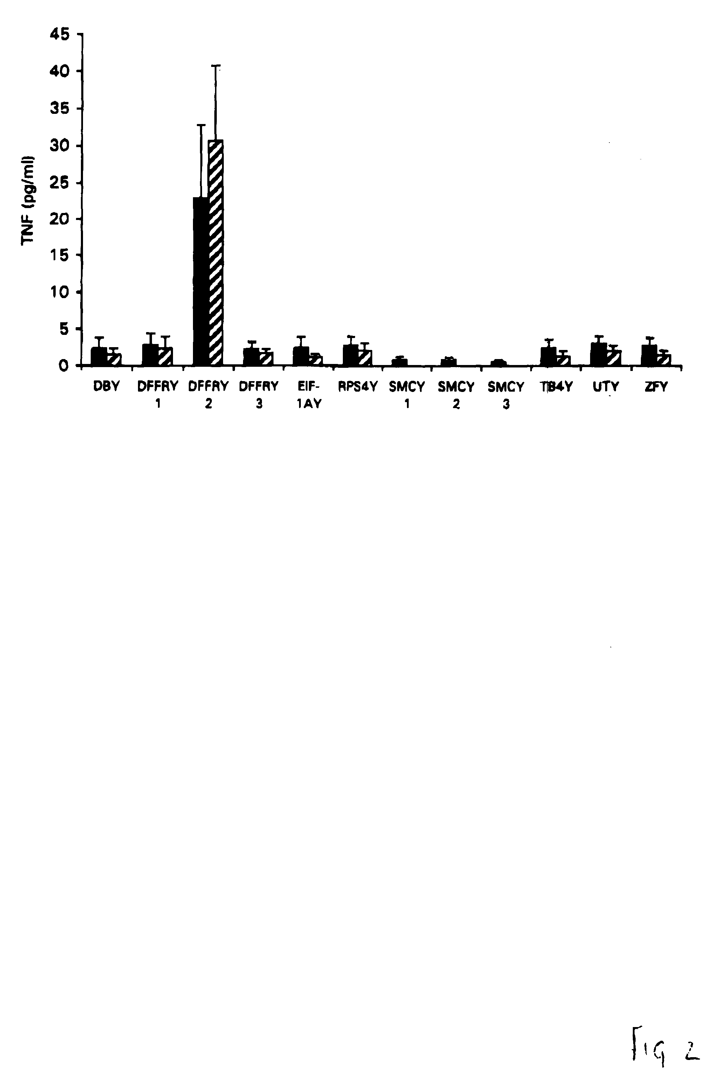

To determine whether one of the Y-specific genes encodes for the HLA-A1 restricted H-Y epitope, the cloned Y-cDNAs were transfected into the HeLa cells stably transfected with HLA-A1 (HeLa/HLA-A1) or cotransfected with HLA-A1 cDNA into HeLa cells. As shown in figure 2, tranfection of DFFRY2 cDNA resulted in a significant amount of TNF production by HLA-A1 HY, whereas transfection of all other Y-genes induced no TNF release. To investigate whether this TNF release by HLA-A1 HY was HLA-A1 restricted, the DFFRY2 cDNA was transfected with and without HLA-A1 into HeLa cells. No significant TNF release by HLA-A1 HY took place when the DFFRY2 gene was transfected in the absence of the HLA-A1 molecule (TNF production 3.9 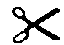 1.5 pg/ml).

### localization of the HLA-A1 associated H-Y epitope in the DFFRY gene

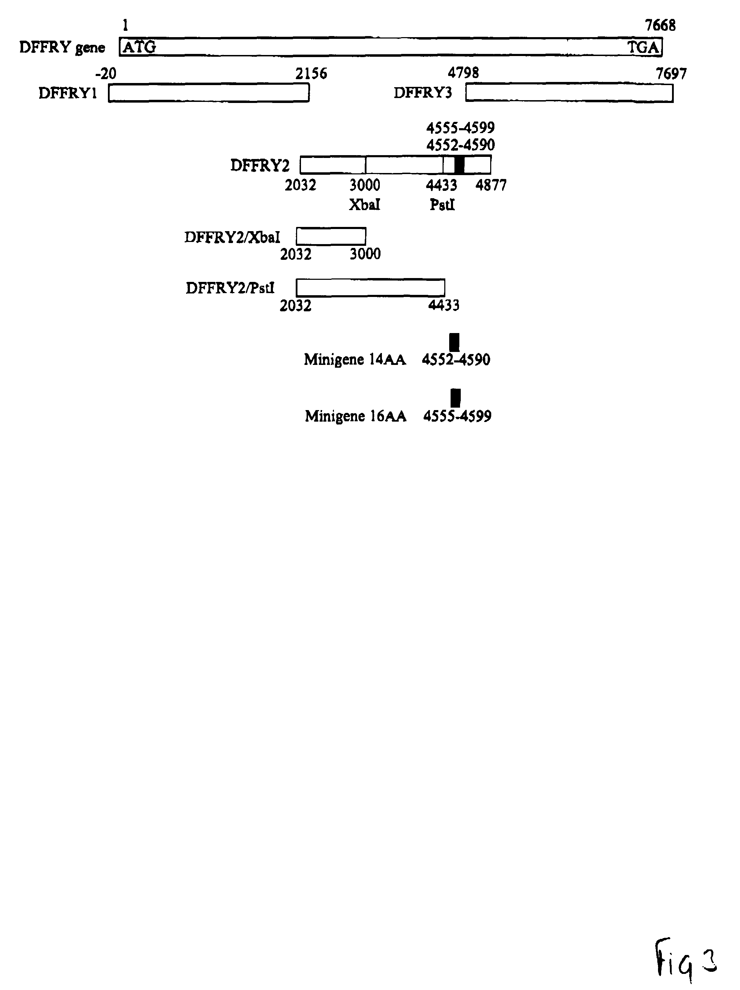

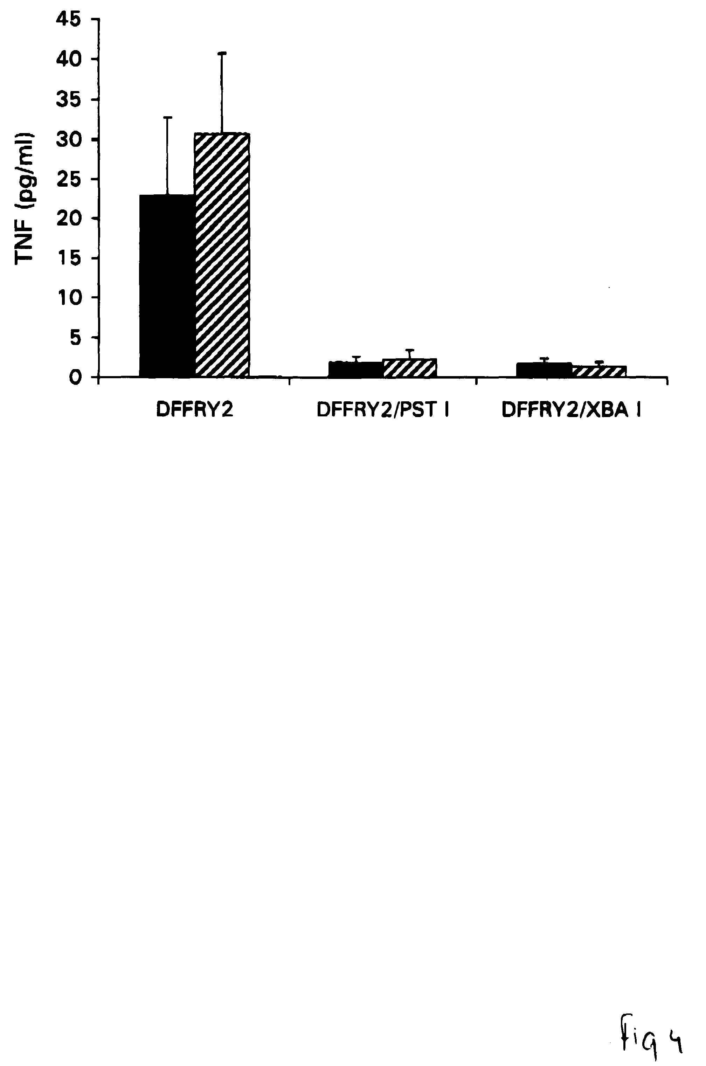

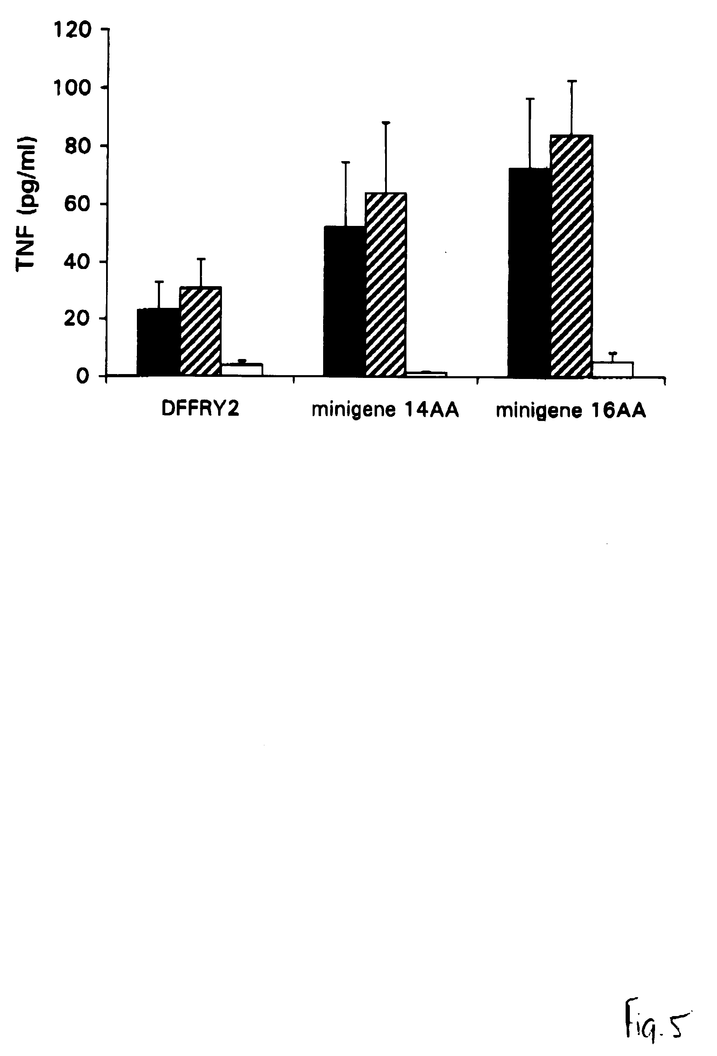

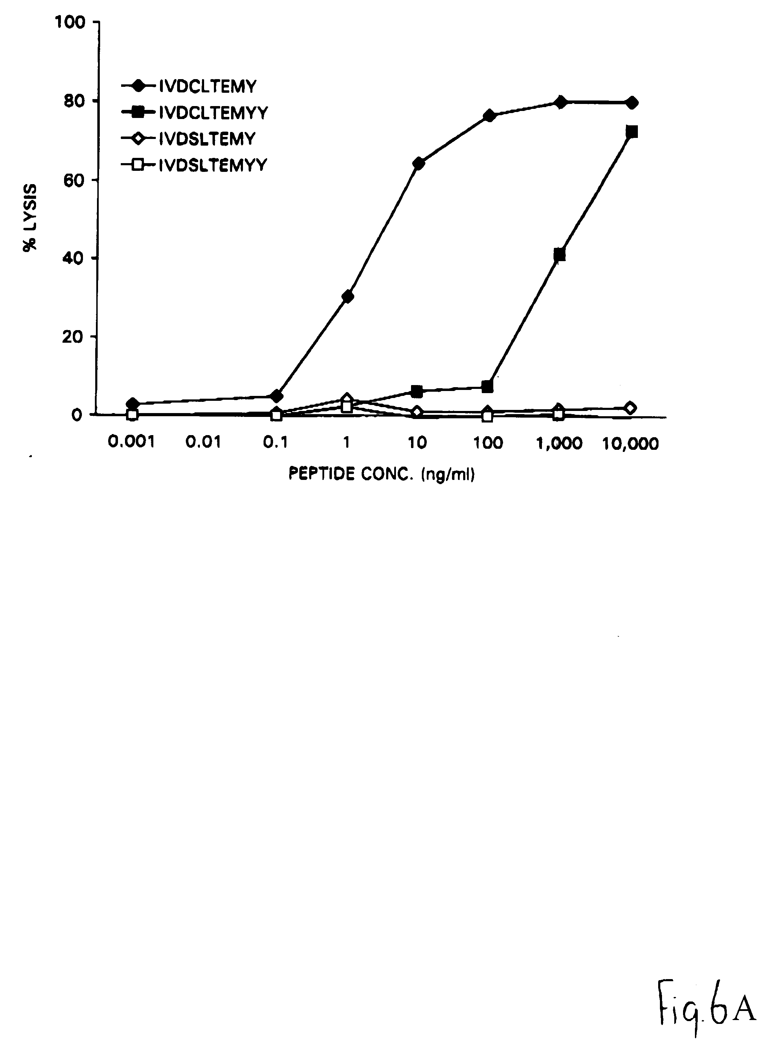

A genetic approach was used to localize the DFFRY sequence coding for the H-Y epitope. First, deletion mutants were obtained by digestion with restriction enzymes PstI and XbaI, resulting in a deletion at the 3' site of the DFFRY2 gene of 444 bp (DFFRY2/PstI) and 1877 bp (DFFRY2/XbaI) respectively (fig. 3). As is demonstrated in figure 4, TNF production by HLA-A1 HY was abolished when the CTL clone was stimulated with HeLa transfected with these DFFRY2 deletion mutants and HLA-A1. We concluded that the HLA-A1 associated H-Y antigenic peptide was encoded by the sequence of the DFFRY gene located between position 4433 and 4828, 30 base pairs after the starting base of DFFRY3. In order to identify the H-Y epitope, the 4433-4828 region of the DFFRY gene was screened for peptides that would bind to HLA-A1 and at least would differ for one amino acid compared to the X-homologue. The DFFRY region 4561 to 4587 or 4590 codes for a HLA-A1 binding nonapetide and decapeptide respectively. To determine whether this region indeed coded for the H-Y epitope, two pairs of oligo nucleotides were synthesized. Each pair of oligo nucleotides consisted of two complementary single stranded DNA (ssDNA), which formed double stranded DNA minigenes with a BamHI site and HindIII site after hybridization. These minigenes were cloned into the BamHI/HindIII digested PCR3.1 expression vector. Minigene 14AA codes for the peptide MLKQIVDCLTEMYY (DFFRY position 4552-4590) and minigene 16AA for MKQIVDCLTEMYYMGT (DFFRY position 4555-4599) (fig. 3). Both minigenes were transfected into HeLa/A1 or cotransfected with HLA-A1 into HeLa. As is demonstrated in figure 5, both minigenes induced TNF production by HLA-A1 HY, indicating that they encoded the H-Y epitope. The TNF production was abolished when no cotransfection of the HLA-A1 molecule into HeLa cells was performed.

### Identification of the HLA-A1 associated H-Y epitope

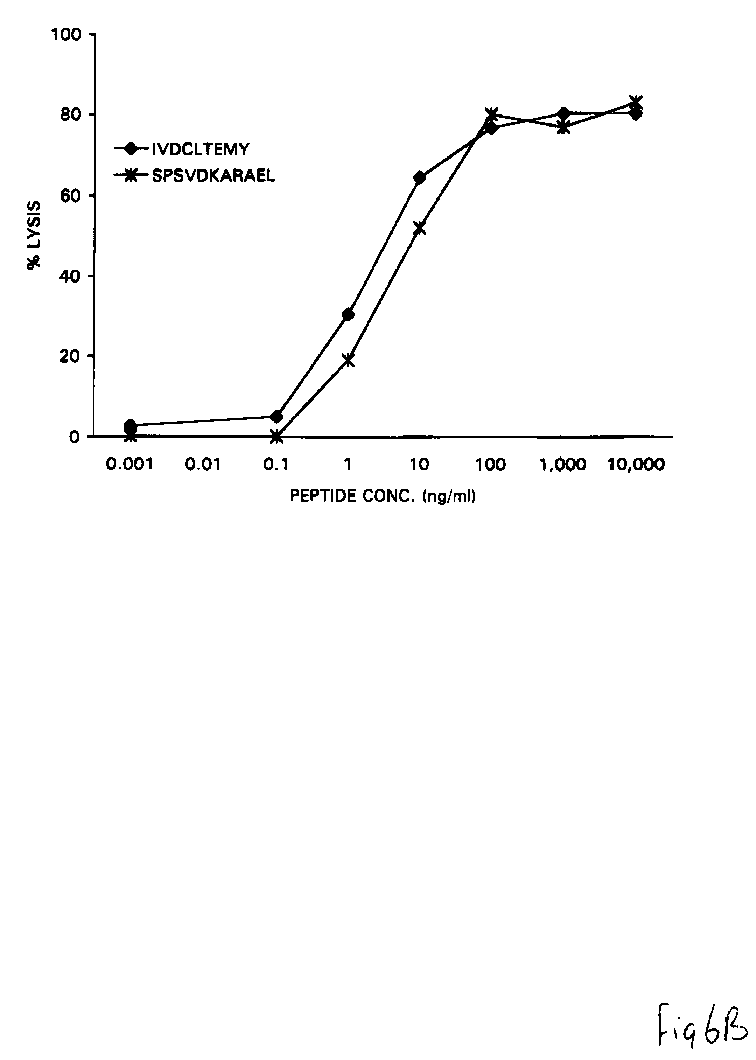

The nonapeptide IVDCLTEMY and decapeptide IVDCLTEMYY encoded by the minigenes were synthesized and loaded at various concentrations on female HLA-A1 positive EBV-LCL cells, derived from the original patient who rejected her male bone marrow graft. The X-homologues IVDSLTEMY and IVDSLTEMYY were used as controls. Recognition of the peptide loaded EBV-LCL cells by the CTL clone HLA-A1 HY was determined in a Cr-release assay. As is shown in figure 6a, the IVDCLTEMY loaded EBV-LCL cells were efficiently killed by HLA-A1 HY with half-maximal target cell lysis at a peptide concentration of 1 ng/ml, whereas the IVDCLTEMYY loaded EBV-LCL cells were only killed at high peptide concentrations. Both X-homologue peptides were not recognized by HLA-A1 HY.
As a control, the previously identified epitope IVDCLTEMY derived from the SMCY protein and recognized by a HLA-B7 restricted H-Y specific CTL clone was loaded at various concentrations on female HLA-B7 positive EBV-LCL cells. Recognition of the peptide loaded EBV-LCL cells by the CTL clone HLA-B7 HY was determined in a Cr-release assay. As is demonstrated in figure 6b, the CTL clone HLA-A1 HY recognized the HLA-A1 associated H-Y epitope IVDCLTEMY with the same dose dependent efficiency as the CTL clone HLA-B7 HY recognized its specific target IVDCLTEMY in the context of HLA-B7.

### Discussion.

Graft rejection or GVHD after HLA-identical stem cell transplantation are thought to be the result of recognition of mH antigens by immunocompetent T lymphocytes from recipient or donor origin, respectively (1,4). The involvement of male-specific mH antigens in graft rejection was identified by the observation that female patients transplanted with HLA-phenotypically identical male bone marrow had a higher risk of developing graft rejection compared to transplants of female origin (9). T lymphocyte clones specifically recognizing male target cells could be generated during graft-rejection after sex-mismatched transplantation, and can be used as tools to identify H-Y antigens and the corresponding Y-specific genes (11,22).

Previously, two human H-Y epitopes have been identified that were involved in bone marrow graft rejection. The HLA-A2 and the HLA-B7 associated H-Y epitope recognized by a HLA-A2 and a HLA-B7 restricted CTL clone, respectively (12,13), were both encoded by the ubiquitously expressed SMCY gene, located in the same Yq deletion interval as the human H-Y antigen controlling locus HY (23,24). We report the identification of a Y-chromosomal gene coding for a new male-specific transplantation antigen in the context of HLA-A1: DFFRY. The DFFRY-derived peptide was recognized by a HLA-A1 restricted CTL clone, which was also generated during graft rejection from a female AML patient who rejected her HLA-phenotypically identical bone marrow derived from her father. The identification of this gene demonstrates that human H-Y antigen consists of at least two genes present on the human Y-chromosome.

DFFRY maps to proximal Yqll.2 within the H-Y antigen controling locus HY (15,25). The complete coding region of DFFRY shows 91% identity at the amino acid level to the X-homologue DFFRX (25). Both Y- and X-linked gene have similarity to the Drosophila fat facets (faf) gene (25). Recently, it has been shown that faf is a member of a family of deubiquitinating genes that play an important regulatory role at the level of protein turnover by preventing degradation of proteins by the proteasome through the removal of conjugated ubiquitin (26). The DFFRY and DFFRX gene contains the conserved Cys and His domains that are characteristic of ubiquitin-specific hydrolases. The high degree of conservation of these domains found in the genes argues strongly that they have a homologous biochemical function. Ubiquitous expression of DFFRX and DFFRY appears to support this view. However, it remains possible that the differences in amino acid sequence between DFFRY and DFFRX may lead to subtle differentiation of biological function.

As was examined by RT-PCR with DFFRY specific primers, the human DFFRY mRNA is expressed in a wide range of tissues, whereas the mouse DFFRY gene is expressed specifically in the testis (25). Consequently, the mouse DFFRY gene cannot encode male-specific transplantation antigens, whereas the human DFFRY gene may also code for male rejection antigens after transplantation of non hematopoietic cells or organs. Previously, it was demonstrated that transplantation of human male donor heart into a female recipient significantly increases the number of graft rejections as compared to sex-matched transplantation (27). Similarly, transplantation of a liver from a male donor into a female recipient increases the occurrence of chronic graft rejection compared to liver transplantation of a female donor (28). These reports indicate that H-Y antigens may be involved in organ allograft rejections as well.

The DFFRY gene codes for a very large protein (>2500 residues) that differs from the female homologue by over 230 residues that are relatively uniformly distributed throughout its length. There is sufficient polymorphism in the DFFRY sequence to allow the generation of different peptides that are both male specific and capable of binding to different MHC molecules. So, DFFRY has at least the same potential to generate a large number of distinct DFFRY-specific H-Y epitopes as compared to SMCY, which differs from the X-homologue by over 200 residues. Identification of other H-Y epitopes will elicit the role of DFFRY as H-Y epitope encoding gene.

The 9 amino acid HLA-A1 associated H-Y epitope derived from the DFFRY gene contains one polymorphism compared to the X-homologue peptide: a cysteine residue at position 4 instead of a serine residue. Recently, a HLA-A2 associated H-Y antigen derived from the SMCY gene was identified which contained a cysteine residue that had undergone a post translational modification that significantly affected T cell recognition (13). This described modification involves attachment of a second cysteine residue to the cysteine in the primary sequence via a disulfide bond. Half-maximal target cell lysis of T2 cells by the specific CTL clone was achieved by using a 100 pM concentration of the cysteinylated peptide, whereas a concentration of 180 nM of the unmodified peptide was required to achieve the same result. Half-maximal target cell lysis of EBV-LCL cells by the DFFRY-specific CTL clone was achieved by using a DFFRY peptide concentration of approximately 1 nM. Although this peptide concentration was comparable to the concentration required for reconstitution of the HLA-B7 associated H-Y epitope derived from the SMCY gene, we do not exclude that cysteinylation of the DFFRY epitope can take place during the Cr-release assay. We used normal RPMI-1640 medium containing 0.21 mM cystine, in which peptide cysteinylation by disulfide exchange with cysteine can occur rapidly.

Characterization of H-Y antigens involved in graft rejection may be useful in selection of donor-recipient combinations. As was shown previously, a strong recipient anti-donor cellular cytotoxic reactivity directed against the DFFRY-derived H-Y antigen in the female AML patient could be demonstrated before transplantation (1). The recipient rejected her bone marrow 60 days after BMT, even after highly immunosuppressive conditioning regimens. The frequency of H-Y specific CD8+ T cells in the female recipient can be determined using HLA-peptide tetrameric complexes (29,30). Determination of the frequency of H-Y specific CD8+ T cells in female recipients by HLA/H-Y peptide tetramerics improves donor selection and allows the determination of bone marrow transplantation recipients at high risk for H-Y-induced graft rejection.

### Figure legends:

Figure 1: In vitro transcription/translation of Y-specific cDNAs. The Y-specific genes were transcribed and translated using Wheat Germ extract and ³H-labeled Leucine. The ³H-labeled proteins were separated on a 12% SDS PAGE gel and visualized by autoradiography.

Figure 2: TNF production by HLA-A1 HY after stimulation with HeLa transfected with Y-specific cDNAs. HeLa cells were cotransfected with each Y-specific cDNA and HLA-A1 cDNA (black bars), or HLA-A1 stably transfected HeLa cells were transfected with each Y-specific cDNA only (hatched bars). 48 hours after transfection, HLA-A1 HY was added. The culture supernatants were harvested 1 day later and tested for TNF production.

Figure 3: Location of DFFRY deletion mutants and HLA-A1 HY epitope-encoding minigenes.

Figure 4: No recognition of DFFRY2 deletion mutants by HLA-A1 HY. HeLa cells were cotransfected with DFFRY2 cDNA and HLA-A1 cDNA (black bars), or HLA-A1 stably transfected HeLa cells were transfected with DFFRY2 cDNA only (hatched bars). 48 hours after transfection, HLA-A1 HY was added. The culture supernatants were harvested 1 day later and tested for TNF production.

Figure 5: Minigenes encode epitope recognized by HLA-A1 HY. HeLa cells were cotransfected with DFFRY2 or minigene cDNA and HLA-A1 cDNA (black bars), or HLA-A1 stably transfected HeLa cells were transfected with DFFRY2 cDNA only (hatched bars). As a control, HeLa cells were transfected with DFFRY2 or minigene cDNA only (white bars). 48 hours after transfection, HLA-A1 HY was added. The culture supernatants were harvested 1 day later and tested for TNF production.

Figure 6: Specific lysis of peptide loaded female EBV-LCL target cells by HLA-A1 HY and HLA-B7 HY. (A) Female recipient HLA-A1 positive EBV-LCL cells were Cr-labeled for 1 hour. After washing, the cells were incubated for 1 hour with DFFRY and DFFRX derived peptides at various concentrations. HLA-A1 HY was added at an effector to target ratio of 10 and Cr release was measured after 4 hours. (B) After Cr-labeling, the female recipient HLA-A1 positive EBV-LCL cells were pulsed with the DFFRY derived peptide IVDCLTEMY and female HLA-B7 positive EBV-LCL cells with the SMCY derived peptide IVDCLTEMY at various concentrations. HLA-A1 HY and HLA-B7 HY were added at an effector to target ratio of 10 and Cr release was measured after 4 hours.

### References

1. Voogt, P. J., W. E. Fibbe, W. A. Marijt, E. Goulmy, W. F. Veenhof, M. Hamilton, A. Brand, F. E. Zwann, R. Willemze, and J. J. van Rood. 1990. Rejection of bone-marrow graft by recipient-derived cytotoxic T lymphocytes against minor histocompatibility antigens. *Lancet* 335:131.
2. Bosserman, L. D., C. Murray, T. Takvorian, K. C. Anderson, A. S. Freedman, J. Fitzsimmons, F. Coral, L. M. Nadler, S. F. Schlossman, and J. Ritz. 1989. Mechanism of graft failure in HLA-matched and HLA-mismatched bone marrow transplant recipients. *Bone Marrow Transplant.* 4:239.
3. Bunjes, D., W. Heit, R. Arnold, T. Schmeiser, M. Wiesneth, F. Carbonell, F. Porzsolt, A. Raghavachar, and H. Heimpel. 1987. Evidence for the involvement of host-derived OKT8-positive T cells in the rejection of T-depleted, HLA-identical bone marrow grafts. *Transplantation* 43:501.
4. Marijt, W. A., N. A. Kernan, T. Diaz-Barrientos, W. F. Veenhof, R. J. O'Reilly, R. Willemze, and J. H. Falkenburg. 1995. Multiple minor histocompatibility antigen-specific cytotoxic T lymphocyte clones can be generated during graft rejection after HLA- identical bone marrow transplantation. *Bone Marrow Transplant.* 16:125.
5. Eichwald, E. J. and C. R. Silmser. 1955. *Transplant. Bull.* 2:148.
6. Baily, D. W. and J. Hoste. 1971. A gene governing the female immune response to the male antigen in mice. *Transplantation* 11:404.
7. Goulmy, E., A. Termijtelen, B. A. Bradley, and J. J. van Rood. 1977. Y-antigen killing by T cells of women is restricted by HLA. *Nature* 266:544.
8. Goulmy, E., A. Termijtelen, B. A. Bradley, and J. J. van Rood. 1976. Alloimmunity to human H-Y [letter]. *Lancet* 2:1206.
9. Bortin, M. M. 1987. Acute graft-versus-host disease following bone marrow transplantation in humans: prognostic factors. Advisory Committee of the International Bone Marrow Transplant Registry. *Transplant. Proc.* 19:2655.
10. Scott, D. M., I. E. Ehrmann, P. S. Ellis, C. E. Bishop, A. I. Agulnik, E. Simpson, and M. J. Mitchell. 1995. Identification of a mouse male-specific transplantation antigen, H-Y [see comments]. *Nature* 376:695.
11. Marijt, W. A., W. F. Veenhof, E. Goulmy, R. Willemze, J. J. van Rood, and J. H. Falkenburg. 1993. Minor histocompatibility antigens HA-1-, -2-, and -4-, and HY-specific cytotoxic T-cell clones inhibit human hematopoietic progenitor cell growth by a mechanism that is dependent on direct cell-cell contact. *Blood* 82:3778.
12. Wang, W., L. R. Meadows, J. M. den Haan, N. E. Sherman, Y. Chen, E. Blokland, J. Shabanowitz, A. I. Agulnik, R. C. Hendrickson, and C. E. Bishop. 1995. Human H-Y: a male-specific histocompatibility antigen derived from the SMCY protein [see comments]. *Science* 269:1588.
13. Meadows, L., W. Wang, J. M. den Haan, E. Blokland, C. Reinhardus, J. W. Drijfhout, J. Shabanowitz, R. Pierce, A. I. Agulnik, C. E. Bishop, D. F. Hunt, E. Goulmy, and V. H. Engelhard. 1997. The HLA-A*0201-restricted H-Y antigen contains a posttranslationally modified cysteine that significantly affects T cell recognition. Immunity. 6:273.
14. King, T. R., G. J. Christianson, M. J. Mitchell, C. E. Bishop, D. Scott, I. Ehrmann, E. Simpson, E. M. Eicher, and D. C. Roopenian. 1994. Deletion mapping by immunoselection against the H-Y histocompatibility antigen further resolves the Sxra region of the mouse Y chromosome and reveals complexity of the Hya locus. *Genomics* 24:159.
15. Lahn, B. T. and D. C. Page. 1997. Functional coherence of the human Y chromosome. *Science* 278:675.
16. Greenfield, A., D. Scott, D. Pennisi, I. Ehrmann, P. Ellis, L. Cooper, E. Simpson, and P. Koopman. 1996. An H-YDb epitope is encoded by a novel mouse Y chromosome gene. *Nat. Genet.* 14:474.
17. Millar, A. D., D. G. Miller, J. V. Garcia, and C. M. Lynch. 1993. Use of retroviral vectors for gene transfer and expression. *Methods in enzymology* 217:581.
18. Kaashoek, J. G., R. Mout, J. H. Falkenburg, R. Willemze, W. E. Fibbe, and J. E. Landegent. 1991. Cytokine production by the bladder carcinoma cell line 5637: rapid analysis of mRNA expression levels using a cDNA-PCR procedure. *Lymphokine Cytokine. Res.* 10:231.
19. Cluitmans, F. H., B. H. Esendam, J. E. Landegent, R. Willemze, and J. H. Falkenburg. 1993. Regulatory effects of T cell lymphokines on cytokine gene expression in monocytes. *Lymphokine Cytokine. Res.* 12:457.
20. Coulie, P. G., V. Brichard, A. Van Pel, T. Wolfel, J. Schneider, C. Traversari, S. Mattei, E. De Plaen, C. Lurquin, and J. P. Szikora. 1994. A new gene coding for a differentiation antigen recognized by autologous cytolytic T lymphocytes on HLA-A2 melanomas [see comments]. *J. Exp. Med.* 180:35.
21. Sugarman, B. J., B. B. Aggarwal, P. E. Hass, I. S. Figari, M. A. Palladino,Jr., and H. M. Shepard. 1985. Recombinant human tumor necrosis factor-alpha: effects on proliferation of normal and transformed cells in vitro. *Science* 230:943.
22. de Bueger, M., A. Bakker, and E. Goulmy. 1992. Existence of mature human CD4+ T cells with genuine class I restriction. *Eur. J. Immunol.* 22:875.
23. O'Reilly, A. J., N. A. Affara, E. Simpson, P. Chandler, E. Goulmy, and M. A. Ferguson-Smith. 1992. A molecular deletion map of the Y chromosome long arm defining X and autosomal homologous regions and the localisation of the HYA locus to the proximal region of the Yq euchromatin. *Hum. Mol. Genet.* 1:379.
24. Cantrell, M. A., J. S. Bogan, E. Simpson, J. N. Bicknell, E. Goulmy, P. Chandler, R. A. Pagon, D. C. Walker, H. C. Thuline, and J. M. Graham, Jr. 1992. Deletion mapping of H-Y antigen to the long arm of the human Y chromosome. *Genomics* 13:1255.
25. Brown, G. M., R. A. Furlong, C. A. Sargent, R. P. Erickson, G. Longepied, M. Mitchell, M. H. Jones, T. B. Hargreave, H. J. Cooke, and N. A. Affara. 1998. Characterisation of the coding sequence and fine mapping of the human DFFRY gene and comparative expression analysis and mapping to the Sxrb interval of the mouse Y chromosome of the Dffry gene. *Hum. Mol. Genet.* 7:97.
26. Huang, Y., R. T. Baker, and J. A. Fischer-Vize. 1995. Control of cell fate by a deubiquitinating enzyme encoded by the fat facets gene. *Science* 270:1828.
27. Kawauchi, M., S. R. Gundry, J. A. de Begona, D. A. Fullerton, A. J. Razzouk, M. M. Boucek, S. Nehlsen-Cannarella, and L. L. Bailey. 1993. Male donor into female recipient increases the risk of pediatric heart allograft rejection [see comments]. *Ann. Thorac. Surg.* 55:716.
28. Candinas, D., B. K. Gunson, P. Nightingale, S. Hubscher, P. McMaster, and J. M. Neuberger. 1995. Sex mismatch as a risk factor for chronic rejection of liver allografts. *Lancet* 346:1117.
29. Ogg, G. S. and A. J. McMichael. 1998. HLA-peptide tetrameric complexes. *Curr. Opin. Immunol.* 10:393.
30. Tan, L. C., N. Gudgeon, N. E. Annels, P. Hansasuta, C. A. O'Callaghan, S. Rowland-Jones, A. J. McMichael, A. B. Rickinson, and M. F. Callan. 1999. A Re-Evaluation of the Frequency of CD8+ T Cells Specific for EBV in Healthy Virus.

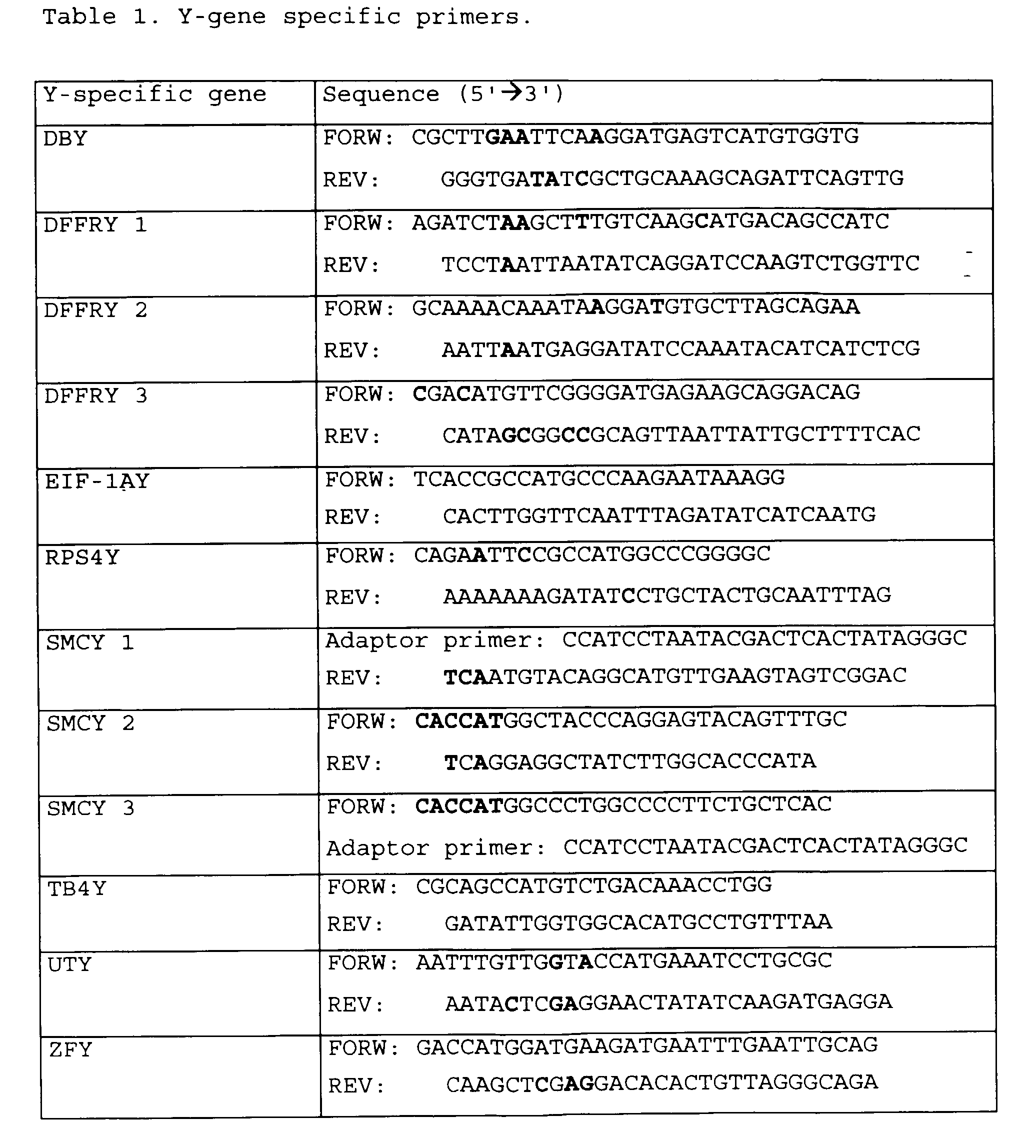

## Claims

1. DFFRY or DFFRX proteins or homologues, derivatives and/or specific fragments thereof for use as a pharmaceutical, in particular a medicament for modifying an immune response in a host.

2. Use of DFFRY or DFFRX proteins or homologues, derivatives and/or specific fragments thereof in the preparation of a medicament for modifying an immune response in a host.

3. Use according to claim 1 or 2, wherein said host is a recipient of donor cells.

4. Use according to claim 1 or 2, wherein said host is a donor-of cells for a recipient.

5. Use according to claim 3 or 4, wherein said donor cells comprise hematopoietic cells.

6. Use according to claim 3 or 4, wherein at least part of said donor cells constitute an organ allograft.

7. Use according to anyone of the aforegoing claims, wherein said medicament induces tolerance for DFFRY or DFFRX proteins or homologues, derivatives and/or specific fragments thereof in said host.

8. Use according to any one of claims 1-6, whereby said medicament at least partially eliminates an immune response to DFFRY or DFFRX proteins or homologues, derivatives and/or specific fragments thereof in said host.

9. Use according to claim 8, whereby said medicament eliminates a subset of T cells.

10. A diagnostic testkit for HLA matching and/or mismatching between at least two individuals, comprising a means for determining the presence or absence of DFFRY or DFFRX proteins or homologues, derivatives and/or specific fragments thereof in a sample.

11. A diagnostic testkit for HLA matching and/or mismatching between at least two individuals, comprising a means for determining the presence or absence of nucleic acids encoding DFFRY or DFFRX proteins or homologues, derivatives and/or specific fragments thereof in a sample.

12. A peptide constituting a T-cell epitope obtainable from a DFFRY or DFFRX protein or homologue thereof.

13. A peptide constituting a T-cell epitope obtainable from a DFFRY or DFFRX protein or homologue thereof comprising the sequence IVDCLTEMY or IVDCLTEMYY or a derivative thereof having similar immunological properties.

14. An immunogenic polypeptide obtainable from a DFFRY or DFFRX protein or homologue thereof comprising the sequence IVDCLTEMY or IVDCLTEMYY or a derivative thereof having similar immunological properties.

15. A vaccine comprising an epitope or a polypeptide according to claim 12,13 or 14.

16. A pharmaceutical formulation comprising an epitope or a polypeptide according to claim 12, 13 or 14.

17. Peptide or polypeptide according to claim 12, 13 or 14 for use as a medicine.

18. Use of a peptide or polypeptide according to claim 12, 13 or 14 in the preparation of a medicament for the induction of tolerance for transplants to prevent rejection and/or Graft versus Host disease.

19. A method for the elimination of a subset of T-cells recognizing a peptide according to claim 12 or 13, wherein said peptide is provided with a toxic moiety.

20. Analogue of the peptide according to claim 12 or 13, which is an antagonist for the activity of T cells recognizing said peptide.

21. Method for the generation of antibodies, T cell receptors, anti-idiotypic B-cells or T-cells, comprising the step of immunisation of a mammal with a peptide or a polypeptide according to claim 12, 13 or 14.

22. Antibodies, T-cell receptors, B-cells or T-cells obtainable by the method of claim 21.


### Annex to the application documents - subsequently filed sequences listing

SEQUENCE LISTING

<110> Rijksuniversiteit te Leiden

<120> A male-specific protein homologue involved in
      histocompatability

<130> P50063EP00

<140> 99201864.8
<141> 1999-06-11

<160> 35

<170> PatentIn Ver. 2.1

<210> 1
<211> 9
<212> PRT
<213> Homo sapiens

<220>
<221> PEPTIDE
<222> (1)..(9)
<223> /Note="T-cell epitope"

<400> 1
Ile Val Asp Cys Leu Thr Glu Met Tyr
  1               5

<210> 2
<211> 10
<212> PRT
<213> Homo sapiens

<220>
<221> PEPTIDE
<222> (1)..(10)
<223> /Note="T-cell epitope"

<400> 2
Ile Val Asp Cys Leu Thr Glu Met Tyr Tyr
  1               5                  10

<210> 3
<211> 9
<212> PRT
<213> Homo sapiens

<220>
<221> PEPTIDE
<222> (1)..(9)
<223> /Note="T-cell epitope"

<400> 3
Ile Val Asp Ser Leu Thr Glu Met Tyr
1               5

<210> 4
<211> 10
<212> PRT
<213> Homo sapiens

<220>
<221> PEPTIDE
<222> (1)..(10)
<223> /Note="T-cell epitope"

<400> 4
Ile Val Asp Ser Leu Thr Glu Met Tyr Tyr
1               5                   10

<210> 5
<211> 54
<212> DNA
<213> Artificial Sequence

<220>
<221> primer_bind
<222> (1)..(54)
<223> /Note="oligo nucleotide for hybridization"

<220>
<223> Description of Artificial Sequence: primer

<400> 5
agcttcacca tgctcaaaca gatagtagac tgtttgactg aaatgtatta
ctag        54

<210> 6
<211> 54
<212> DNA
<213> Artificial Sequence

<220>
<221> primer_bind
<222> (1)..(54)
<223> /Note="oligo nucleotide for hybridization"

<220>
<223> Description of Artificial Sequence: primer

<400> 6
gatcctagta atacatttca gtcaaacagt ctactatctg tttgagcatg
gtga        54

<210> 7
<211> 60
<212> DNA
<213> Artificial Sequence

<220>
<221> primer_bind
<222> (1)..(60)
<223> /Note="oligo nucleotide for hybridization"

<220>
<223> Description of Artificial Sequence: primer

<400> 7
agcttcacca tgaaacagat agtagactgt ttgactgaaa tgtattacat
gggcacatag 60


<210> 8
<211> 60
<212> DNA
<213> Artificial Sequence

<220>
<221> primer_bind
<222> ()..)
<223> /Note="oligo nucleotide for hybridization"

<220>
<223> Description of Artificial Sequence: primer

<400> 8
gatcctatgt gcccatgtaa tacatttcag tcaaacagtc tactatctgt
ttcatggtga 60


<210> 9
<211> 14
<212> PRT
<213> Homo sapiens

<220>
<221> PEPTIDE
<222> (1)..(14)
<223> /Note="T-cell epitope"

<400> 9
Met Leu Lys Gln Ile Val Asp Cys Leu Thr Glu Met Tyr Tyr
1               5                   10


<210> 10
<211> 16
<212> PRT
<213> Homo sapiens

<220>

<221> PEPTIDE
<222> (1)..(16)
<223> /Note="T-cell epitope"

<400> 10
Met Lys Gln Ile Val Asp Cys Leu Thr Glu Met Tyr Tyr Met
Gly Thr
  1               5                   10
15

<210> 11
<211> 30
<212> DNA
<213> Artificial Sequence

<220>
<221> primer_bind
<222> (1)..(30)
<223> /Note="Y-gene specific primer (forward)"

<220>
<223> Description of Artificial Sequence: primer

<400> 11
cgcttgaatt caaggatgag tcatgtggtg
30

<210> 12
<211> 31
<212> DNA
<213> Artificial Sequence

<220>
<221> primer_bind
<222> (1)..(31)
<223> /Note="Y-gene specific primer (reverse)"

<220>
<223> Description of Artificial Sequence: primer

<400> 12
gggtgatatc gctgcaaagc agattcagtt g
31

<210> 13
<211> 32
<212> DNA
<213> Artificial Sequence

<220>
<221> primer_bind
<222> (1)..(32)
<223> /Note="Y-gene specific primer (forward)"

<220>
<223> Description of Artificial Sequence: primer

<400> 13
agatctaagc tttgtcaagc atgacagcca tc
32



<210> 14
<211> 32
<212> DNA
<213> Artificial Sequence

<220>
<221> primer_bind
<222> (1)..(32)
<223> /Note="Y-gene specific primer (reverse)"

<220>
<223> Description of Artificial Sequence: primer

<400> 14
tcctaattaa tatcaggatc caagtctggt tc
32



<210> 15
<211> 30
<212> DNA
<213> Artificial Sequence

<220>
<221> primer_bind
<222> (1)..(30)
<223> /Note="Y-gene specific primer (forward)"

<220>
<223> Description of Artificial Sequence: primer

<400> 15
gcaaaacaaa taaggatgtg cttagcagaa
30



<210> 16
<211> 32
<212> DNA
<213> Artificial Sequence

<220>
<221> primer_bind
<222> (1)..(32)
<223> /Note="Y-gene specific primer (reverse)"

<220>
<223> Description of Artificial Sequence: primer

<400> 16

aattaatgag gatatccaaa tacatcatct cg
32

<210> 17
<211> 30
<212> DNA
<213> Artificial Sequence

<220>
<221> primer_bind
<222> (1)..(30)
<223> /Note="Y-gene specific primer (forward)"

<220>
<223> Description of Artificial Sequence: primer

<400> 17
cgacatgttc ggggatgaga agcaggacag
30

<210> 18
<211> 32
<212> DNA
<213> Artificial Sequence

<220>
<221> primer_bind
<222> (1)..(32)
<223> /Note="Y-gene specific primer (reverse)"

<220>
<223> Description of Artificial Sequence: primer

<400> 18
catagcggcc gcagttaatt attgcttttc ac
32

<210> 19
<211> 25
<212> DNA
<213> Artificial Sequence

<220>
<221> primer_bind
<222> (1)..(25)
<223> /Note="Y-gene specific primer (forward)"

<220>
<223> Description of Artificial Sequence: primer

<400> 19
tcaccgccat gcccaagaat aaagg
25

<210> 20
<211> 29
<212> DNA
<213> Artificial Sequence

<220>
<221> primer_bind
<222> (1)..(29)
<223> /Note="Y-gene specific primer (reverse)"

<220>
<223> Description of Artificial Sequence: primer

<400> 20
cacttggttc aatttagata tcatcaatg
29


<210> 21
<211> 24
<212> DNA
<213> Artificial Sequence

<220>
<221> primer_bind
<222> (1)..(24)
<223> /Note="Y-gene specific primer (forward)"

<220>
<223> Description of Artificial Sequence: primer

<400> 21
cagaattccg ccatggcccg gggc
24


<210> 22
<211> 30
<212> DNA
<213> Artificial Sequence

<220>
<221> primer_bind
<222> (1)..(30)
<223> /Note="Y-gene specific primer (reverse)"

<220>
<223> Description of Artificial Sequence: primer

<400> 22
aaaaaaagat atcctgctac tgcaatttag
30


<210> 23
<211> 27
<212> DNA
<213> Artificial Sequence

<220>
<221> primer_bind
<222> (1)..(27)
<223> /Note="Y-gene specific primer (adaptor primer)"

<220>
<223> Description of Artificial Sequence: primer

<400> 23
ccatcctaat acgactcact atagggc
27


<210> 24
<211> 31
<212> DNA
<213> Artificial Sequence

<220>
<221> primer_bind
<222> (1)..(31)
<223> /Note="Y-gene specific primer (reverse)"

<220>
<223> Description of Artificial Sequence: primer

<400> 24
tcaatgtaca ggcatgttga agtagtcgga c
31


<210> 25
<211> 29
<212> DNA
<213> Artificial Sequence

<220>
<221> primer_bind
<222> (1)..(29)
<223> /Note="Y-gene specific primer (forward)"

<220>
<223> Description of Artificial Sequence: primer

<400> 25
caccatggct acccaggagt acagtttgc
29


<210> 26
<211> 25
<212> DNA
<213> Artificial Sequence

<220>
<221> primer_bind
<222> (1)..(25)

<223> /Note="Y-gene specific primer (reverse)"

<220>
<223> Description of Artificial Sequence: primer

<400> 26
tcaggaggct atcttggcac ccata
25

<210> 27
<211> 28
<212> DNA
<213> Artificial Sequence

<220>
<221> primer_bind
<222> (1)..(27)
<223> /Note="Y-gene specific primer (forward)"

<220>
<223> Description of Artificial Sequence: primer

<400> 27
caccatggcc ctggcccctt ctgctcac
28

<210> 28
<211> 27
<212> DNA
<213> Artificial Sequence

<220>
<221> primer_bind
<222> (1)..(27)
<223> /Note="Y-gene specific primer (adaptor primer)"

<220>
<223> Description of Artificial Sequence: primer

<400> 28
ccatcctaat acgactcact atagggc
27

<210> 29
<211> 24
<212> DNA
<213> Artificial Sequence

<220>
<221> primer_bind
<222> (1)..(24)
<223> /Note="Y-gene specific primer (forward)"

<220>
<223> Description of Artificial Sequence: primer

<400> 29
cgcagccatg tctgacaaac ctgg
24

<210> 30
<211> 26
<212> DNA
<213> Artificial Sequence

<220>
<221> primer_bind
<222> (1)..(26)
<223> /Note="Y-gene specific primer (reverse)"

<220>
<223> Description of Artificial Sequence: primer

<400> 30
gatattggtg gcacatgcct gtttaa
26

<210> 31
<211> 28
<212> DNA
<213> Artificial Sequence

<220>
<221> primer_bind
<222> (1)..(28)
<223> /Note="Y-gene specific primer (forward)"

<220>
<223> Description of Artificial Sequence: primer

<400> 31
aatttgttgg taccatgaaa tcctgcgc
28

<210> 32
<211> 30
<212> DNA
<213> Artificial Sequence

<220>
<221> primer_bind
<222> (1)..(30)
<223> /Note="Y-gene specific primer (reverse)"

<220>
<223> Description of Artificial Sequence: primer

<400> 32
aatactcgag gaactatatc aagatgagga
30

<210> 33
<211> 31
<212> DNA
<213> Artificial Sequence

<220>
<221> primer_bind
<222> (1)..(31)
<223> /Note="Y-gene specific primer (forward)"

<220>
<223> Description of Artificial Sequence: primer

<400> 33
gaccatggat gaagatgaat ttgaattgca g
31

<210> 34
<211> 29
<212> DNA
<213> Artificial Sequence

<220>
<221> primer_bind
<222> (1)..(29)
<223> /Note="Y-gene specific primer (reverse)"

<220>
<223> Description of Artificial Sequence: primer

<400> 34
caagctcgag gacacactgt tagggcaga
29

<210> 35
<211> 11
<212> PRT
<213> Homo sapiens

<220>
<221> PEPTIDE
<222> (1)..(11)
<223> /Note="T-cell epitope"

<400> 35
Ser Pro Ser Val Asp Lys Ala Arg Ala Glu Leu
1               5                   10